# EUROPEAN PATENT APPLICATION

(11) **EP 1 238 653 A1**
(43) Date of publication of application: **11.09.2002**
(21) Application number: 01200879.3
(22) Date of filing: 09.03.2001
(51) Int. Cl.: A61K 7/48, A61K 7/50

(54) **Skin care products with improved skin and material softness**

(71) Applicant: JOHNSON & JOHNSON GmbH, 40474 Düsseldorf (DE)
(72) Inventor: Lange, Rainer, 53608 Bad Honnef (DE); Martens, Nicolas, 53604 Bad Honnf (DE)
(74) Representative: Wante, Dirk

(57) **Abstract**

This invention relates to products comprising a porous or absorbent sheet, in particular a wipe, and a liquid, in particular an oil-in-water emulsion, wherein the liquid contains glyceryl monooleate.

## Description

### Background of the Invention

This invention relates to products comprising a porous or absorbent sheet, in particular a wipe, and a liquid, in particular an oil-in-water emulsion, wherein the liquid contains glyceryl monooleate.

Wipe products have become an important product category that has found a wide variety of applications for adults and babies. Examples include face or body cleansing wipes, wipes for skin treatment or skin conditioning.

Over the last couple of decades so-called wet wipes have become successful as products particularly suited for these applications. These products are typically manufactured by impregnating sheets made of non-woven fabric with a suitable lotion.

Recent innovations in the wipes area included improvements in the fabric, in the impregnating liquid as well as in product presentation. Initially, wet wipe products were made of traditional non-woven materials based on paper making technology (pulp based products). These products were well accepted but deficient in softness of the fabric material. The introduction of the 'spunlace' non-woven technology offered products that were superior in terms of the softness compared to traditional paper based products. This is mainly due to (i) the use of long soft fibres (most frequently rayon and PET / PP or a mixture of these fibres) in the spunlace process and (ii) the fact that during the spunlace process no binder is added to the fabric.

Another innovation was the introduction of the 'Pop-up' technology that offered advantages as regards the dispensing of individual wipes.

Irrespective of its end use the softness of the wipe product is of primary importance to the consumer. Softness of the wipe material on the one hand, and perceived softness of the skin after usage of the wipe on the other, are important consumer benefits. This in particular is the case for applications on babies.

Almost all wipe products currently on the market are based on non-wovens as the fabric material. However, since non-wovens are manufactured in large scale processes which are relatively inflexible and also require high capital investment it is difficult and often not attractive to further improve the softness of the original substrate.

A second approach in improving the softness of non-woven fabrics is to add fabric softeners to either the finished product or to the fibres used as raw materials. This approach has been taken in many applications of dry non-wovens. Particularly in dry facial tissue products and toilet paper the softness has been significantly improved via the addition of 'fabric softeners'. Most of these softeners are silicon-based compounds or derivatives thereof.

In spite of these improvements there is still a need to improve softness of wipe products and to provide wipe products that result in the skin having a softer feel after application of the wipe. Providing such products is an object of this invention.

This object is attained by the products provided herein which have a positive effect on the softness of the fabric as well as on the softness of skin after use. Although as mentioned above, the addition of softeners has been used intensively in dry wipe products it has been found that the agents used in the products according to the invention also improve the softness in wet wipe applications.

### Summary of the Invention

The present invention concerns products comprising a porous or absorbent sheet and a composition that contains glyceryl monooleate. Preferably the composition is liquid and is coated onto or impregnated into said sheet. Alternatively, the composition or the liquid additionally contains a C₈₋₂₀ alkyl glucoside, in particular coco-glycoside.

In a particular embodiment, the composition is liquid and more in particular is an emulsion which preferably is an oil in water emulsion, more preferably an oil in water emulsion prepared according to the phase inversion technique.

In an alternative aspect this invention concerns the use of glyceryl monooleate or of a combination of the latter with a C₈₋₂₀ alkyl glucoside, in particular with coco-glycoside, or the use of a composition containing said monooleate or said combination, to improve the softness of a porous or absorbent sheet.

Or alternatively there is provided a method of improving the softness of a porous or absorbent sheet, which method comprises applying glyceryl monooleate or of a combination of the latter with a C₈₋₂₀ alkyl glucoside, in particular with coco-glycoside, or applying a composition containing said monooleate or said combination, to said sheet. In a preferred aspect the composition in said use or method is liquid or is an emulsion as described herein.

In a further aspect the invention concerns the use of a product as defined herein to improve skin softness. Or alternatively, the invention concerns a method to improve skin softness which comprises applying a product as defined herein to the skin.

### Detailed Description of the Invention

Whenever used in this description and claims, any percentage is weight by weight (w/w).

The sheet of absorbent or porous material for use in the products of this invention can take the form of a tissue, a wipe, towel, towelette, and the like.

Sheet materials that can be used can be mono or multi-layered, woven or non-woven. They can be made of one or of several materials. Particularly preferred are non-woven materials that have a web structure of fibrous or filamentous nature, in which the fibres or filaments are distributed randomly or with a certain degree of orientation, the former being obtainable by air-laying or certain wet-laying processes, the latter in other wet-laying or in carding processes. The fibres or filaments can be natural, for example wood pulp, wool cotton, linen and the like, or synthetic, for example polyvinyls, polyesters, polyolefins, polyamides and the like.

Typically they have a weight per square meter in the range of 10 to 80 g/m2, in particular of 20 to 70 g/m2. Particular materials are of the non-woven type. Based on the raw material that has been used, two different types of products can be distinguished.

A first type of carriers is paper based. The raw materials for these carriers are made almost exclusively of cellulose-based fibres or filaments from plant cellular sources (pulp). These can be available from fresh wood-shavings or from recycled material (recycled paper). In a number of wipe applications, such as baby wipes, wipes for cleansing, wet paper towels and the like, high wet strength or firmness of the non-woven web is a desirable attribute. This can be achieved by the addition of binding materials. Examples of such materials are the so-called wet strength resins. In some cases additives are added in order to increase the softness of the end product.

In a second type use the web is made mainly of staple, e.g. based on cotton, wool, linen and the like.

Commercial products are made of cellulose fibres, synthetic fibres or mixtures of both. Polyester and polypropylene are known as suitable polymers for the preparation of synthetic fibres. Also in these products binders can be used to increase the firmness of the non-woven fabric.

Webs of increased strength can be obtained by using the so-called spunlace or hydro-entanglement technique. In this technique the individual fibres are twisted together so that an acceptable strength or firmness is obtained without using binding materials. The advantage of the latter technique is the excellent softness of the non-woven material.

Non woven materials that are made of a mixture of pulp and staple are also known. Such materials are available with binding materials, in particular those mentioned above, or without binding materials. In the latter instance the non-woven is preferably made by the spunlace or hydro-entaglement procedure.

In a preferred embodiment of the present invention, the carrier material is made of cellulose pulp with a small amount of binding material. The amount of binder in the carrier material is in the range of 5 to 20 % (w/w).

In a particularly preferred embodiment the non-woven fabric is prepared by the water entanglement procedure and does not contain binding material.

The absorbing ability of the carrier material is of particular interest with regard to the applications envisaged by the present invention. During production the impregnating solution should be taken up quickly by the carrier. In certain embodiments of this invention the wipes will be packed in a stack of a plurality of wipes. In this instance the absorbing ability of the non-woven fabric should be such that a chromatographic effect (sinking down of the lotion) in the stack is avoided during storage. On the other hand it should be guaranteed that during the usage of the wipe the lotion is delivered evenly to the skin.

The absorbing capacity of the carrier material is determined essentially by three different parameters: the surface weight of the carrier material, the nature of the raw material used in the manufacture and the manufacturing process used.

For the applications according to the invention the carrier materials typically have a surface weight from 10 g/m² to 80 g/m², preferably from 30 to 70 g/m² and more preferably from 40 to 60 g/m². The selection of the raw material of which the non-woven carrier is made depends on the manufacturing procedure. Typically in the manufacture of non-woven carriers by the hydro-entanglement process, use is made of mixtures of cellulose fibres and synthetic fibres. The relative quantity of synthetic fibres in the non-woven fabric is from 0 to 100 % and preferably is between 10 and 70 %, more preferably in the range of 30 to 50% (all percentages being w/w).

The products of the present invention further comprise a composition containing glyceryl monooleate, preferably a composition containing glyceryl monooleate and a C₈₋₂₀ alkyl glucoside, in particular coco-glucoside. In particular, these compositions are liquid compositions, preferably they are emulsion-based. These liquid compositions, which also are referred to as 'lotions', preferably are of aqueous nature.

The emulsions can be oil in water or water in oil emulsions, or be of more complex nature such as water-in-oil-in-water. Preferred are oil in water emulsions, more in particular oil in water emulsions prepared according to the phase inversion technique.

The compositions for use in the products of the invention contain glyceryl monoolate. The amount of the latter in particular is in the range from 0.01 to 2 %, in particular from 0.015 to 1 %, preferably from 0.0175 to 0.5 %, more preferably in the range from 0.0175 to 0.335 %, or from 0.02 to 0.5 % still more preferably from 0.08 to 0.2 %.

In a particular embodiment the compositions for use in the products of the invention additionally contain a fatty alcohol glucoside, in particular a C₈₋₂₀ alkyl glucoside, more in particular a C₈₋₁₆ alkyl glucoside, preferably coco-glucoside. The amount of said glucoside in the composition in particular is in the range from 0.01 to 2 %, in particular from 0.015 to 1 %, preferably from 0.0175 to 0.5 %, more preferably in the range from 0.0175 to 0.5 %, or from 0.02 to 0.335 % still more preferably from 0.08 to 0.2 %.

All percentages in this and the preceding paragraph are w/w percentages.

As used herein C₈₋₂₀ alkyl or C₈₋₂₀ alkyl refers to straight or branch chained hydrocarbon radicals, saturated or unsaturated, having from about 8 to about 20 or from about 8 to about 16 carbon atoms, including mixtures thereof. C₈₋₂₀ alkyl or C₈₋₁₆ alkyl in particular is derived from fatty alcohols. Examples of C₈₋₁₆ alkyl are capryl, 2-ethylhexyl, caprinyl, lauryl, isotridecyl, myristyl, palmoleyl, cetyl, and the like. C₈₋₂₀ alkyl comprises these radicals as well as stearyl, isostearyl, oleyl, linolenyl, linolyl, and the like.

The ratio of the amount of glyceryl monoolate to the fatty alcohol glucoside in the compositions for use in the products of the invention is in the range from 2 : 1 to 1 : 2, preferably in the range from 1.5 : 1 to 1 : 1.5, most preferably said ratio is about 1 : 1.

A particularly suited combination is that which is sold under the trademark 'Lamesoft', in particular 'Lamesoft PO65', a mixture of 20 to 40 % of glyceryl monooleate, 20 to 40 % of coco glucoside and water. This 'Lamesoft' product is added to the compositions in an amount in the range from 0.1 to 1 %, preferably from 0.1 to 0.5 %, more preferably from 0.25 to 0.5 %.

The amount of the composition on the wipe will be in the range from about 100 to about 400 %, preferably from about 200 % to about 400 %, expressed as the weight of the composition relative to the weight of the sheet in dry condition.

Preferred compositions are those based on emulsions prepared by the so-called phase inversion technique. The phase inversion technique is described in more detail by F. Förster, F. Schambil, and H. Tesmann in Int. J. Cos. Sci. 1990 (12) 217.

According to this technique, oil-in-water formulations made with non-ionic emulsifiers typically undergo a phase inversion upon heating which means that within a particular temperature interval a change of the emulsion type takes place, i.e. from an oil-in-water to a water-in-oil emulsion. In this process the external continuous phase changes from being aqueous to an oily phase resulting in a drop of the electrical conductivity to virtually zero. The average temperature between that of maximal and of minimal conductivity is referred to as the phase inversion temperature ('PIT').

After heating to a temperature above the PIT, the emulsion is cooled below the PIT whereupon the inverse phase transfer takes place, i.e. from water-in-oil to oil-in-water. The resulting emulsions are usually referred to as 'PIT emulsions'.

The droplet size of the PIT emulsion depends on a number of factors. PIT emulsions with small droplet size can be obtained with emulsions forming microemulsions having a low surface tension between the oil and water phases at the phase inversion, or that form a laminar liquid crystalline phase.

Preferred are PIT emulsions that are finely dispersed, i.e. having a small droplet size and have low viscosity.

The oily phase in PIT emulsions comprises natural oils or natural oil derivatives, in particular of vegetal origin. Examples are linseed oil, palm oil, olive oil, castor oil, rapeseed oil, soja oil, and in particular peanut oil, coconut oil, sunflower oil and turnip seed oil. The oily phase may further comprise fatty components isolated from these natural oils, i.e. pure triglycerides or mixtures thereof, or the latter components having been prepared chemically. These so-called trigycerides are esters of glycerine with fatty acids or fatty acid mixtures. Preferred triglycerides are those glycerine esters derived from fatty acids, either saturated or unsaturated, having from 10 to 24, particularly from 14 to 20, preferably from 16 to 18 carbon atoms, for example palmitic, heptadecanoic, oleic or stearic acid, or mixtures thereof. Particularly preferred is glyceryl stearate, also referred to as stearin.

The oily phase may further comprise alkyl esters of fatty acids, wherein the alkyl group has from 1 to 4 carbon atoms. Preferred are C₁₋₄ alkyl esters of C₁₆₋₁₈ fatty acids, for example of palmitic, heptadecanoic, or stearic acid, in particular the methyl or ethyl esters, including mixtures thereof.

Of particular interest are oily phases that comprise a vegetable oil or a triglyceride in combination with an alkyl ester of a fatty acid.

The PIT emulsion further contains a non-ionic emulsifier.

Suitable non-ionic emulsfiers comprise:
polyethoxylated or propoxylated fatty alcohols, fatty acids or C ₈₋₁₅ alkylphenols, having 2 to 30 ethoxy units and 0 to 5 propoxy units, or 1 to 5 propoxy units, prepared by reacting the starting alcohols with ethylene or propylene oxide;
mono- or diesters of polyethoxylated glycerine that with saturated or unsaturated C₁₂₋₁₈ fatty acids, having 1 to 30 ethoxy units;
glycerin mono- or diesters and sorbitan mono- or diesters of saturated or unsaturated fatty acids as well as ethoxylated derivatives thereof, the latter in particular having from 1 to 30 ethoxy units;
C ₈₋₂₂ alkyl mono- or oligoglycosides as well as ethoxylated derivatives thereof, the latter in particular having from 1 to 30 ethoxy units;
ethoxylated castor oil or hydrogenated castor oil, in particular having from 1 to 30 ethoxy units;
polyol fatty acid esters and in particular polyglycerine fatty acid esters, more in particular ricinoelic acid or hydroxy stearic acid esters; for example polyglycerine poly ricinoleic acid or polyglycerine poly 12-hydroxystearate; and mixtures thereof;
glycerine, polyglycerine, mono- and di-pentaerythrite, sugar derived alcohols such as sorbitol, alkylglucosides and polyglucosides, partially esterified with one ore more fatty acids or fatty acid mixtures;
trialkylphosphates as well as polyethoxylated derivatives thereof, the latter in particular having from 1 to 30 ethoxy units;
wool wax alcohols;
polysiloxane-polyalkyl-polyether copolymers and derivatives thereof;
mixed ethers of pentaerythrite, fatty acids, citric acid and fatty alcohols polyalkylene glycols;
glycerine carbonate.

As used herein the term fatty acid refers to saturated or unsaturated, straight or branch chained alkanoic acids, optionally substituted with one or more hydroxy groups.

Particular useful emulsifiers comprise an emulsifier system containing a mixture of a hydrophilic and hydrophobic emulsifier.

Hydrophilic emulsifiers comprise ethoxylated fatty alcohols or fatty acids. Examples of the former are ethoxylated C₁₆₋₂₂ alcohols such as for example cetyl, palmoleyl, stearyl, isostearyl and oleyl alcohol and mixtures thereof wherein the number of ethoxyl groups per molecule is in the range of 1 to 35, preferably from 1 to 20, more preferably from 10 to 20.

Examples of ethoxylated fatty acids are ethoxylated C₁₂₋₂₂ alkylcarbonic acids such as, for example, palmitinic, palmoleinic, steraic, isosearic acid and mixtures thereof, wherein the number of ethoxy groups is in the range of 5 to 50, in particular from 15 to 35.

Hydrophobic emulsifiers comprise polyethoxylated glycerin fatty acid mono- and diesters having 1 to 30 ethoxy units, i.e. polethoxylated glycerin wherein between 1 and 2 of the hydroxy functions have been esterified with 1 or 2 fatty acids or fatty acid mixtures.

The w/w ratio of the hydrophilic emulsifier components to the hydrophobic emulsifier components is in the range of 10 : 90 to 90 : 10, in particular 25 :75 to 75:25, more in particular in the range of 40 : 60 to 60 : 40.

The PIT emulsions for use in the products according to the invention in particular contain from 20 to 90 %, more in particular from 30 to 80 % and preferably 30 to 60 % of water. The remainder making up the formulation comprises the oily phase, the emulsifiers and other components. The oily phase typically comprises from 10 to 80 %, in particular from 40 to 70 % of the formulation. Preferred are emulsion wherein the w/w ratio of the oil and aqueous phases are about 1:1. The emulsifiers are present in an amount that is in the range of 1 to 25 %, in particular 5 to 20 % and more in particular 5 to 15 %.

The phase inversion temperature typically is in the range from 20 to 95 °C, in particular in the range from 40 to 95 °C.

The PIT lotions for use in the present invention will contain one or more light absorbing or light reflecting substances, in particular those mentioned herein. These can be hydrophilic or hydrophobic. In the former instance these substances will be solved into the aqueous phase while in the latter into the oily phase.

Particular PIT emulsions that can be used in the sunscreen compositions of this invention are described for example in WO-00/51427 and in WO-00/71676

The compositions prepared by the phase inversion technique preferably have a viscosity of below 100 mPas. The average particle size of the oil droplets is in the range of 50 to 300 nm, in particular in the range of 50 to 200 nm, and preferably is 100 nm or smaller, e.g. between 70 and 90 nm. These compositions are particularly attractive in that they show good spreading and impregnating properties.

The compositions for use in the products of the invention may further contain skin caring and/or active ingredients like emollients, oils, plant extracts, vitamins, etc. Oils can be of natural or synthetic origin, e.g. vegetable oils or mineral oils or the group of silicones.

The group of emollients comprises lipids like lanolin, lanolin alcohols, lanolin acids, polyethoxylated or acylated lanolin or lanolin derivatives, lecithin and lecithin derivatives, fatty alcohols , either linear or branched with chain lengths between C6 and C40, and their esters with organic acids, e.g. carbonic acids or polyacids containing between 2 and 30 C atoms, branched, aromatic or linear including hydroxy or amino acids, fatty acids and fatty acid esters with alcohols or poly alcohols containing between 2 and 40 C atoms, branched, aromatic or linear, sterols found in the unsaponifiable fraction of e.g. avocado oil, almond oil, soybean oil, etc. like soy phytosterol, β-sitosterol, β-sitosteryl laurate, β-sitosteryl stearate, etc. natural and synthetic waxes, e.g. bees wax, purcelline, shea butter, cocoa butter, ceresin, ozokerit, vaseline,micro wax, carnauba wax candelilla wax and alike, substituted cyclohexanes like di-n-octylcyclohexane, Guerbet carbonates, e.g. bis-2-octyl dodecylcarbonate, dialkyl ethers like di-n-octyl ether, etc.

Examples of oils are natural oils, e.g. almond oil, soybean oil, wheat germ oil, avocado oil, jojoba oil, linseed oil, sesame oil, walnut oil, sunflower oil, olive oil, etc., mineral and paraffin oil and synthetic oils comprising mono-, di-, triglycerides as well as mixtures thereof.

Silicones comprise e.g. dimethyl polysiloxanes, methylphenyl polysiloxanes, cyclic silicones and silicon compounds derivatised with amino-, fatty acid, alcohol-, polyether, epoxy-, and/or alkyl groups.

The compositions may also contain film-forming substances like chitosan and derivatives thereof, derivatives of poly acrylic acid, polyvinyl pyrrolidone and its derivatives, etc.

The compositions may further contain active ingredients such as anti-microbials (anti-bacterials and antifungals), anti inflammatory agents, anti irritating compounds, sunscreen agents, moisturising agents, anti-wrinkle agents, plant extracts, vitamines, and the like. Examples of such ingredients comprise complexes of PVP and hydrogen peroxide, diclofenac, acetyl salicylic acid, salicylates, ibuprofen, bisabolol, mimosa extract (mimosa tenuiflora), hyaluronic acid, propylene glycol, glycerin, chondroitin sulfate, plant extracts, bisabolol, panthenol, tocopherol, actives for anti-stinging, anti-irritants, anti-dandruffs, anti-ageing agents e.g. retinol, melibiose etc. Other suitable actives are e.g. medicago officinalis, actinidia chinensis, allantoin, aloe barbadensis, anona cherimolia, anthemis nobilis, arachis hypogaea, arnica montana, avena sativa, beta-carotene, bisabolol, borago officinalis, butylene glycol, calendula officinalis, camellia sinensis, camphor, candida bombicola, capryloyl glycine, carica papaya, centaurea cyanus, cetylpyridinium chloride, chamomilla recutita, chenopodium quinoa, chinchona succirubra, chondrus crispus, citrus aurantium dulcis, citrus grandis, citrus limonum, cocos nucifera, coffea arabica, crataegus monogina, cucumis melo, dichlorophenyl imidazoldioxolan, enteromorpha compressa, equisetum arvense, ethoxydiglycol, ethyl panthenol, farnesol, ferulic acid, fragaria chiloensis, gentiana lutea, ginkgo biloba, glycerin, glyceryl laurate, glycyrrhiza glabra, hamamelis virginiana, heliotropine, hydrogenated palm glycerides, citrate, hydrolyzed castor oil, hydrolyzed wheat protein, hypericum perforatum, iris florentina, juniperus communis, lactis proteinum, lactose, lawsonia inermis, linalool, linum usitatissimum, lysine, magnesium aspartate, magnifera indica, malva sylvestris, mannitol, mel, melaleuca alternifolia, mentha piperita, menthol, menthyl lactate, mimosa tenuiflora, nymphaea alba, olaflur, oryza sativa, panthenol, paraffinum liquidum, PEG-20M, PEG-26 jojoba acid, PEG-26 jojoba alcohol, PEG-35 castor oil, PEG-40 hydrogenated castor oil, PEG-60 hydrogenated castor oil, PEG-8 caprylic/capric acid, persea gratissima, petrolatum, potassium aspartate, potassium sorbate, propylene glycol, prunus amygdalus dulcis, prunus armeniaca, prunus persica, retinyl palmitate, ricinus communis, rosa canina, rosmarinus officinalis, rubus idaeus, salicylic acid, sambucus nigra, sarcosine, serenoa serrulata, simmondsia chinensis, sodium carboxymethyl betaglucan, sodium cocoyl amino acids, sodium hyaluronate, sodium palmitoyl proline, stearoxytrimethylsilane, stearyl alcohol, sulfurized TEA-ricinoleate, talc, thymus vulgaris, tilia cordata, tocopherol, tocopheryl acetate, trideceth-9, triticum vulgare, tyrosine, undecylenoyl glycine, urea, vaccinium myrtillus, valine, zinc oxide, zinc sulfate.

The products according to the invention can be made by coating the said composition onto or impregnating it into the sheet material. The term coating also comprises techniques such as printing and spraying of the composition on the sheet. In a preferred embodiment, wipes are impregnated with a lotion prepared according to the PIT procedure.

The compositions for use in the products of the invention are prepared by conventional methods. In a particular embodiment a concentrate is made which subsequently is diluted with a suitable aqueous medium to obtain the composition which is applied to the sheet. In a particular embodiment the concentrate contains glyceryl monooletate and optionally also alkyl glucoside and other components, e.g. one or more emulsifiers, one or more preservatives, fragrances arid the like. The concentrate may also be formulated as an emulsion, in particular as an oil-in-water emulsion, more in particular as an oil-in-water emulsion prepared according to the PIT technique. In that instance the concentrate will contain appropriate emulsifiers, in particular those mentioned herein.

The concentration of glyceryl monooletate and optionally also alkyl glucoside and other components in the concentrate may be from about 2.5 to about 10 times, preferably from about 5 to about 7.5 times higher than the concentration of thereof in the end formulation.

Hence the invention concerns a process for preparing a product as defined herein, said process comprising contacting a suitable carrier with a composition as described herein, in particular with a composition containing glyceryl monooleate. In particular said process comprises impregnating a wipe with a lotion as described herein, more in particular impregnating or spraying a wipe with a sunscreen lotion. The latter preferably is a PIT formulation as described herein.

In a particular execution, the carrier material is cut into strips the transversal size of which being similar to the size of the sheet, in particular the tissue or wipe. Subsequently the carrier strips are folded according to methods generally known and applied in the art. The thus folded strips are moistened with a liquid composition as defined herein, in particular with a PIT composition, said moistening preferably comprising spraying or dripping. Or the fabric strips can first be moistened and subsequently be folded.

The strips can also be impregnated with the composition by immersing in or running the strip through a bath containing the composition. They can also be sprayed or printed with the composition.

In a further step, the strips are cut so that the desired size of the sheets, in particular of the wipes, is obtained. The thus obtained sheets (or wipes) can be packed individually or can be stacked in a determined number, e.g. a number between 10 and 30, preferably between 15 and 25, most preferably about 20, and the stack then packed in a suitable package, for example a plastic wrap, box and the like.

The products according to the invention can take the form of baby or adult wipes and can be used in a wide range of applications as personal care products, comprising, for example, baby cleansing wipes, face or body cleansing wipes, wipes for skin treatment or skin conditioning such as for example skin moisturization, insect repellent wipes, sunscreen wipes, and the like.

The products of the present invention have superior softness, feel and cleansing properties. Softness is the tactile sensation perceived when the consumer holds the product, rubs it across the skin, or crumples it with the hand.

The products show increased softness of the sheet material, more specifically they show increased softness of the non-woven fabric. Additionally, they result in an improvement of the softness of the skin in that it has a softer feel or an improved sensorial softness after application of the wipe. They further result in increased skin smoothness and skin elasticity.

As used herein applying or application to the skin comprises any action contacting the product to the skin e.g. by rubbing across the skin, bathing, dabbing, wetting and the like.

The products of the invention, when applied to the skin, cause a sufficient or an effective amount of glyceryl monooletate and/or of C₈₋₂₀ alkyl glucoside to be released and thereby administered to the skin. The same applies equally to products wherein the composition contains one or more other active ingredients, i.e. these are also caused to be released and therby administered in a sufficient or effective amount. Additionally the present products allow an even distribution of these agents or active ingredients onto the skin.

The glyceryl monooletate and/or of C₈₋₂₀ alkyl glucoside ingredients in the compositions described herein cover or coat the fibres of the fabric. This is particularly the case when using a PIT composition. A fabric being impregnated or coated with a composition as described herein can be allowed to dry resulting in the fabric's fibres being coated or covered by glyceryl monooletate and/or of C₈₋₂₀ alkyl glucoside.

The softness of sheet products can be demonstrated by a number of tests. One such test comprises mounting the sheet to a longitudinal plate at one end of which an object of defined mass is placed. Subsequently that end of the plate is lifted until the object starts gliding downwardly. The angle of the plate at the moment where the weight starts gliding is measured and compared with that of standard sheets.

Softness of the skin can be measured by standardized consumer panel tests in sensory evaluation tests. It can also be quantified in a torque test in which a cylindrical object is pressed against the skin with a defined force and subsequently the object is subjected to a defined torque force and the angle by which the object can be turned is measured.

Skin softness can also be determined by in vivo skin topometry in which the skin surface profile is measured. The rougher the skin the more irregular the skin's surface topology will be.

### Examples

As used in these examples 'Nipaguard IPF' is a trademark for a preseravtive containing 10 % of iodopropyl butylcarbamate (IPBC).
'Emulgade SE-PF™^{,} is Glyceryl stearate/ Ceteareth 20 ™ / Ceteareth 12 ™ / Cetearyl alcohol/ cetyl palmitate mixture
'Eumulgin B2 ™^{,} is Ceteareth 20 ™;
'Ceteareth-20 ™^{,} is ethoxylated cetostearyl alcohol having 20 ethoxy units;
'Ceteareth-12 ™^{,} is ethoxylated cetostearyl alcohol having 12 ethoxy units;
'Nervanaid B30 ™^{,} is tetrasodium EDTA (chelating agent).

### Example 1

| Preparation of a concentrate | |
|---|---|
| Emulgade SE-PF™ | 8,300% |
| Eumulgin B1™ | 4,700% |
| Mineral Oil | 15,000% |
| Phenoxyethanol | 0,830% |
| Nipaguard IPF™ | 0,500% |
| | |
| Fragrance | 0,550% |
| | |
| Aqua | 68,870% |
| Lamesoft P065™ | 1,250% |

The above ingredients are mixed and subsequently slowly warmed above the PIT temperature. This is determined by measuring the electrical conductivity of the mixture. Subsequently the mixture is allowed to cool slowly to room temperature. The thus obtained PIT formulation is has the appearance of a clear solution with low viscosity.

### Example 2

| | |
|---|---|
| Aqua | 78,886% |
| Nervanaid B30™ | 0,200% |
| | |
| Concentrate of example 1 | 20,000% |
| | |
| Premix | |
| Phenoxyethanol | 0,834% |
| Cetylpyridinium chloride | 0,050% |
| Citric Acid Monohydrate | 0,030% |

Nervanaid and water are mixed and to this mixture there is added the required quantity of the concentrate as prepared in example 1. Subsequently a premix of phenoxyethanol and CPC is made and this is added to the mixture. Finally, citric acid is added.

### Example 3

Dry hydro-entangled carrier material made of fabric having a surface weight of 50 g/m2 was cut into strips. The strips were sprayed in the conventional manner with the liquid as prepared in example 2. Liquid addition was set at 6 g per wipe. Subsequently the strips were folded and cut.

## Claims

1. A product comprising a porous or absorbent sheet and a composition that contains glyceryl monooleate.

2. A product according to claim 1 wherein the composition additionally contains a C₈₋₂₀ alkyl glucoside.

3. A product according to claim 2 wherein the composition additionally contains coco-glycoside.

4. A product according to any of claims 1 to 3 wherein the composition is a liquid which is coated onto or impregnated into said sheet.

5. A product according to any of claims 1 to 4 wherein the composition is an emulsion.

6. A product according to claim 5 wherein the emulsion is an oil in water emulsion.

7. A product according to claim 6 wherein the oil in water emulsion is prepared according to the phase inversion technique.

8. A product according to any of claims 1 to 7 wherein the composition contains glyceryl monoolate in an amount which is in the range from 0.01 to 2 %.

9. A product according to any of claims 2 to 8 wherein the composition for contains a C₈₋₂₀ alkyl glucoside in an amount in the range from from 0.01 to 2 %.

10. A product according to claim 9 wherein the ratio of the amount of glyceryl monoolate to the C₈₋₂₀ alkyl glucoside is in the range from 2 : 1 to 1 : 2.

11. A product accoridng to claim 10 wherein the ratio of the amount of glyceryl monoolate to the C₈₋₂₀ alkyl glucoside is about 1:1.

12. The use of glyceryl monooleate or of a combination of the latter with a C₈₋₁₆ alkyl glucoside to improve the softness of a porous or absorbent sheet.

13. The use of a product as claimed in any of claims 1 to 11 to improve skin softness.
